(19) Europäisches Patentamt / European Patent Office / Office européen des brevets

(11) **EP 4 151 223 A1**

(12) **EUROPEAN PATENT APPLICATION**
published in accordance with Art. 153(4) EPC

(43) Date of publication:
**22.03.2023 Bulletin 2023/12**

(21) Application number: **21788534.2**

(22) Date of filing: **16.04.2021**

(51) International Patent Classification (IPC):
***A61K 31/7048*** *(2006.01)* ***A61K 36/185*** *(2006.01)*
***A61P 9/00*** *(2006.01)* ***A61P 15/12*** *(2006.01)*

(52) Cooperative Patent Classification (CPC):
**A61K 31/7048; A61K 36/185; A61P 9/00; A61P 15/12**

(86) International application number:
**PCT/JP2021/015706**

(87) International publication number:
**WO 2021/210673 (21.10.2021 Gazette 2021/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.04.2020 JP 2020074187**

(71) Applicant: **Kao Corporation**
**Chuo-ku**
**Tokyo 103-8210 (JP)**

(72) Inventors:
- **OTSUKA, Mayumi**
  **Haga-gun, Tochigi 321-3497 (JP)**
- **NAGAMORI, Natsumi**
  **Haga-gun, Tochigi 321-3497 (JP)**
- **OHMINAMI, Hideo**
  **Tokyo 131-8501 (JP)**
- **KITAMURA, Naoya**
  **Tokyo 131-8501 (JP)**
- **YAMAMOTO, Naoki**
  **Tokyo 131-8501 (JP)**

(74) Representative: **Vossius & Partner**
**Patentanwälte Rechtsanwälte mbB**
**Siebertstraße 3**
**81675 München (DE)**

Remarks:
The complete document including Reference Table(s) and the Sequence Listing(s) can be downloaded from the EPO website

(54) **AGENT FOR IMPROVING SYMPTOMS OF MENOPAUSE**

(57) Provided is an agent for improving symptoms of menopause which is useful to improve symptoms of menopause. The agent for improving symptoms of menopause comprises delphinidin-3-rutinoside, cyanidin-3-rutinoside, or a combination thereof as an active ingredient.

Fig. 3

Fr. E-3=delphinidin-3-glucoside(1)
Fr. E-4=delphinidin-3-rutinoside(2)
Fr. E-5=cyanidin-3-glucoside(3)
Fr. E-6=cyanidin-3-rutinoside(4)

Fr. E-6
Fr. E-5
Fr. E-4
Fr. E-3
4-H
6'-H
2'-H
5'-H
8-H
6-H
-OH
2',6'-2H



Processed by Luminess, 75001 PARIS (FR)

## Description

Field of the Invention

The present invention relates to an agent for improving symptoms of menopause.

Background of the Invention

In female menopause, various physical complaints such as general malaise, dizziness, headache, sleeplessness, a rush of blood to head, burning sensation, perspiration, palpitation, nausea, diarrhea, shoulder stiffness, lower back pain, and frequent urination occur as the indefinite complaint. Among them, hot flashes (a rush of blood to head, burning sensation) and perspiration are characteristic symptoms of menopause and are seen in many women.

The symptoms of menopause are considered to be due to the reduction in the secretion of female hormone (estrogen) accompanying with aging. Thus, general treatment for the symptoms of menopause is the hormone replacement therapy (HRT). In HRT, estrogen, or estrogen and progesterone are administered.

However, it is said that the symptoms of menopause are intricately associated with not only the reduction in estrogen, but also social and psychological factors due to work, home environment, or the like. In addition, it has been pointed out that HRT has negative aspects such as an increase in the incidence of breast cancer and uterine cancer, and thus, it is required to improve the indefinite complaint of menopause more safely.

Meanwhile, cassis (scientific name: *Ribes nigrum*) is a plant classified into the family of Saxifragaceae, the genus of Ribes and also called blackcurrant, and contains four types of anthocyanin in a large amount. Among cassis anthocyanins, delphinidin-3-rutinoside and cyanidin-3-rutinoside which are rutinoside forms are characteristic components of cassis anthocyanin. It is reported that cassis anthocyanin has an effect of improving blood flow, ameliorates shoulder stiffness, general malaise, and the like (Patent Literature 1), and has an estrogen effect (Non Patent Literature 1).

However, it has not been apparent whether cassis anthocyanin has an effect of improving the symptoms of menopause.

(Patent Literature 1) WO 2001/01798

(Non Patent Literature 1) Molecules, 23(1), 74, 2018

Summary of the Invention

The present invention relates to the following 1) to 7) .

1) An agent for improving symptoms of menopause comprising delphinidin-3-rutinoside, cyanidin-3-rutinoside, or a combination thereof as an active ingredient.

2) A food product for improving symptoms of menopause comprising delphinidin-3-rutinoside, cyanidin-3-rutinoside, or a combination thereof as an active ingredient.

3) Use of delphinidin-3-rutinoside, cyanidin-3-rutinoside, or a combination thereof for producing an agent for improving symptoms of menopause.

4) Use of delphinidin-3-rutinoside, cyanidin-3-rutinoside, or a combination thereof for producing a food product for improving symptoms of menopause.

5) Delphinidin-3-rutinoside, cyanidin-3-rutinoside, or a combination thereof for use in improving symptoms of menopause.

6) A non-therapeutic use of delphinidin-3-rutinoside, cyanidin-3-rutinoside, or a combination thereof for improving symptoms of menopause.

7) A method for improving symptoms of menopause, the method comprising administering or ingesting an effective amount of delphinidin-3-rutinoside, cyanidin-3-rutinoside, or a combination thereof to or by a subject in need thereof.

Brief Description of Drawings

[Figure 1] Figure 1 is a graph indicating the flow of cassis anthocyanin purification.

[Figure 2] Figure 2 is a chromatogram of preparative HPLC of cassis anthocyanin.

[Figure 3] Figure 3 is $^{1}H$ and $^{13}C$-NMR spectra of Fr. E-3 to E-6.

Detailed Description of the Invention

The present invention relates to provision of an agent for improving symptoms of menopause which is useful to improve the symptoms of menopause.

The present inventors found that delphinidin-3-rutinoside and cyanidin-3-rutinoside have an effect of improving the symptoms of menopause such as hot flashes (a rush of blood to head, burning sensation) and perspiration.

According to the agent for improving symptoms of menopause of the present invention, the symptoms of menopause such as hot flashes (a rush of blood to head, burning sensation) and perspiration can be improved.

In the present invention, delphinidin-3-rutinoside is a glycoside in which rhamnosyl-(($\alpha$1$\rightarrow$6)-glucose is bonded to a hydroxy group at position 3 of delphinidin, and has the following structure.

In the present invention, cyanidin-3-rutinoside is a glycoside in which rhamnosyl-($\alpha$1$\rightarrow$6)-glucose is bonded to a hydroxy group at position 3 of cyanidin, and has the following structure.

Delphinidin-3-rutinoside and cyanidin-3-rutinoside may be a salt or a solvate, and both of which are included. These may be used singly or in combination of two or more thereof. Above all, it is preferred to use delphinidin-3-rutinoside in terms of easily obtaining the desired effect.

Delphinidin-3-rutinoside and cyanidin-3-rutinoside can be obtained by chemical synthesis, or by extraction and purification from natural products containing these, in particular, plants. Also, those commercially available as reagents may be used. Since delphinidin-3-rutinoside and cyanidin-3-rutinoside are found in cassis (scientific name: Ribes nigrum), those derived from a cassis extract are preferably used. The cassis extract may also be used as delphinidin-3-rutinoside and cyanidin-3-rutinoside.

The part of cassis to be used is not particularly limited, and may be the whole plant, leaves, stems, flowers, germs, buds, pericarps, fruits, roots, rhizomes, seeds, or a combination thereof. It is preferred to use fruits or pericarps due to the rich content of cassis anthocyanin. Cassis may be used raw, dried, or processed. Examples of the processing include cutting, shredding, grinding, and pulverization.

The extraction method may be appropriately selected, and any of immersion, decoction, leaching, reflux extraction, supercritical extraction, ultrasonic extraction, microwave extraction, and the like may be used.

As the solvent for extraction, either polar solvents or non-polar solvents may be used. Specific examples of the solvent include water; monohydric, dihydric, and polyhydric alcohols; ketones such as acetone and methyl ethyl ketone; esters such as methyl acetate and ethyl acetate; chain or cyclic ethers such as diethyl ether and tetrahydrofuran; polyethers such as polyethylene glycol; saturated or unsaturated hydrocarbons such as hexane; aromatic hydrocarbons such as benzene and toluene; halogenated hydrocarbons such as dichloromethane, chloroform, dichloroethane, and carbon tetrachloride; pyridines; dimethyl sulfoxide; acetonitrile; carbon dioxide, supercritical carbon dioxide; fats/oils, waxes, and other oils; and mixtures thereof. Suitable examples thereof include alcohols and aqueous solutions thereof. Examples of alcohols include methanol, ethanol, 1,3-butylene glycol, n-propanol, isopropanol, n-butanol, isobutanol, sec-butanol, and t-butanol, and preferred examples thereof include ethanol and an aqueous ethanol solution.

The amount of the solvent used in the extraction and the extraction conditions are not particularly limited, as long as sufficient extraction can be carried out, and the amount and the extraction conditions can be appropriately controlled.

The cassis extract may be a crude purified product as long as the crude purified product conforms to acceptable standards for food or pharmaceuticals, contains cassis anthocyanin, and exerts the effect of the present invention. If necessary, treatment such as removal of inactive impurities, deodorization, and decolorization may be carried out by a known technique such as liquid-liquid distribution, solid-liquid distribution, membrane filter, activated carbon, synthetic adsorption resin, ion exchange resin, and decantation.

The purity thereof may be increased by appropriately combining further known separation and purification methods. Examples of the purification means include organic solvent precipitation, centrifugation, ultrafiltration membrane, high performance liquid chromatography, and column chromatography.

The cassis extract may be used as it is, or may be used as a diluted solution diluted with an appropriate solvent, or may be a concentrated extract, a dried powder, or a preparation in paste form. The cassis extract may also be freeze dried and used by being diluted with a solvent usually used in the extraction, such as water, ethanol, or a mixed liquid of water and ethanol, when used. The cassis extract may also be used by being included in a vesicle such as liposome, a microcapsule, or the like.

The cassis extract contains delphinidin-3-rutinoside and cyanidin-3-rutinoside in an amount such that a total amount thereof is preferably 0.1% by mass or more, and more preferably 10% by mass or more.

As shown in the Examples described below, hot flashes and perspiration which are the symptoms of menopause are improved by oral ingestion of delphinidin-3-rutinoside and cyanidin-3-rutinoside. It has been reported that cassis anthocyanin has an estrogen effect (Molecules, 23(1), 74, 2018), and since the estrogen effect of cassis anthocyanin is about 1/1000 of that of estradiol (ibid.), the cassis anthocyanin used in this human efficacy test is considered to have an estrogen effect which is about 1/10 of that of the hormone replacement therapy (HRT). Thus, it is considered that delphinidin-3-rutinoside and cyanidin-3-rutinoside improve the symptoms of menopause without activation of estrogen.

In the meantime, it has been verified that, when delphinidin-3-rutinoside and cyanidin-3-rutinoside are brought into contact with a transformed cell (TRPV4 expression cell) in which TRPV4 is functionally expressed by human TRPV4 gene introduction in the presence of a TRPV4 agonist, delphinidin-3-rutinoside and cyanidin-3-rutinoside have an effect of suppressing the activity of TRPV4 that the amount of intracellular cation ($Ca^{2+}$) influx due to the TRPV4 agonist is suppressed (Reference Example 1).

TRPV4 (transient receptor potential cation channel subfamily V member 4) is one of the proteins which constitute thermosensitive TRP channel. TRPV4 expresses in a wide variety of tissues such as kidney, lung, bladder, heart, skin, brain, and digestive tract, and is considered to function as a sensor molecule which senses various physical and chemical stimuli such as osmotic pressure change, temperature change, shear stress caused by blood flow, and organ volume change, and transmits those stimuli to cells.

Since chlorogenic acid, coffeic acid, and ferulic acid which are known to have an effect of suppressing the activity of TRPV4 have been reported to improve the autonomic nervous function and indefinite complaint (JP-B-6075830, JP-B-5931633, and JP-B-4077149), it is considered that the indefinite complaint of women in menopause can be improved

by suppressing the activity of TRPV4. Thus, delphinidin-3-rutinoside and cyanidin-3-rutinoside are inferred to improve the symptoms of menopause through suppression of the activity of TRPV4.

Therefore, delphinidin-3-rutinoside, cyanidin-3-rutinoside, or a combination thereof can be an agent for improving symptoms of menopause, can be used to improve the symptoms of menopause, and can be used for producing the agent for improving symptoms of menopause.

Here, the "use" is use in a human or a non-human animal, and may be therapeutic use or non-therapeutic use. The "non-therapeutic" is a concept that does not include medical activities, that is, a concept that does not include methods of surgery, treatment, or diagnosis of a human, more specifically, a concept that does not include methods of surgery, treatment, or diagnosis of a human implemented by a physician or a person instructed by a physician.

The Japan Society of Obstetrics and Gynecology defines the symptoms of menopause and menopausal disorders as "the menopause refers to a period of 5 years before and after menopause, and among a wide variety of symptoms which appear during this period, the symptoms not resulting from the structural change are referred to as the symptoms of menopause, and among these symptoms, the pathological condition which disrupts the daily life is referred to as the menopausal disorder" (The guidebook on Women's health care, menopausal medicine, 2014 edition, edited by the Japan Society for Menopause and Women's Health).

Examples of the symptoms of menopause include dizziness, headaches, sleeplessness, a rush of blood to head, burning sensation, perspiration, palpitation, nausea, constipation, diarrhea, shoulder stiffness, lower back pain, joint pain, frequent urination, drying of the skin and mucous membranes, and eczema. As used herein, "the improvement of the symptoms of menopause" means the improvement of the symptoms of menopause, which is preferably the improvement of hot flashes and perspiration.

As used herein, the "hot flashes" refers to sudden consciousness of heat sensation (a rush of blood to head, burning sensation) which occurs on the head, breast, or entire body during menopause.

The "improvement" refers to recovery of the symptoms or conditions, prevention or delay of the worsening of the symptoms or conditions, or reversal, prevention, or delay of the progress of the symptoms or conditions.

The agent for improving symptoms of menopause of the present invention itself becomes a medicament, quasi-drug, or food product for a human or an animal which exerts the action and effect of improving the symptoms of menopause, or may be a material or preparation used by being blended in the medicament, quasi-drug, or food product.

The food product includes foods with functional claims, foods for specified health uses, supplements, and the like which are based on the concept of improving the symptoms of menopause and in which, if necessary, the concept is displayed. These food products are foods for which functional claims are permitted, so that these food products can be distinguished from general food products.

The medicament (including quasi-drug) contains delphinidin-3-rutinoside, cyanidin-3-rutinoside, or the combination thereof as an active ingredient for improving the symptoms of menopause. Further, the medicament may contain, if necessary, a pharmaceutically acceptable carrier, or other active ingredients, pharmacologic components, or the like, as long as the function of the active ingredient is not lost.

Examples of the dosage form of the medicament (including quasi-drug) include oral solid preparations such as a tablet (including a chewable tablet and the like), a capsule, a granule, a powder, and a troche; oral liquid preparations such as an internal liquid and a syrup; and parenteral administration preparations such as an injection, a suppository, an inhalant, a percutaneous absorption agent, and an external agent. Preferred route of administration is oral administration.

The preparation in such a dosage form can be prepared by appropriately combining cassis anthocyanin with a pharmaceutically acceptable carrier (such as an excipient, a binder, a thickener, a disintegrant, a surfactant, a lubricant, a dispersing agent, a buffer, a preservative, a flavoring agent, a fragrance, a coating agent, and a diluent), other medicinal components, and the like in accordance with a common method.

The food product contains delphinidin-3-rutinoside, cyanidin-3-rutinoside, or the combination thereof as the active ingredient for improving the symptoms of menopause. The form of the food product may be solid, semi-solid, or liquid (e.g., drinks). Examples of the food product include drinks such as soft drinks, tea-based drinks, coffee drinks, fruit drinks, carbonated drinks, jelly drinks, and near-water drinks; various food products, for example, foods and drinks as well as nutrients such as jelly, wafers, biscuits, bread, noodles, and sausages; and raw materials thereof. Alternatively, the food product may be a supplement in the form of an oral administration preparation, such as a tablet, a capsule, a granule, a powder, a liquid, or a syrup.

The food product can be prepared by appropriately combining delphinidin-3-rutinoside, cyanidin-3-rutinoside, or the combination thereof with an optional food material, or other active ingredients, or acceptable additives in food product (e.g., a solvent, a softener, an oil, an emulsifying agent, a preservative, an acidulant, a sweetener, a bittering agent, a pH regulator, a stabilizer, a colorant, an ultraviolet absorber, an antioxidant, a moisturizing agent, a thickener, a fixing agent, a dispersing agent, a fluidity improving agent, a wetting agent, a fragrance, a seasoning, and a flavor regulator) in accordance with a common method.

The content of delphinidin-3-rutinoside, cyanidin-3-rutinoside, or the combination thereof in the above medicament, quasi-drug, or food product may vary depending on their dosage forms or forms. For example, in the case of a food product or an oral administration preparation in a solid form such as a tablet, a granule, a pill, a powder, or a gummy, the total amount of delphinidin-3-rutinoside and cyanidin-3-rutinoside is preferably 0.1% by mass or more, more preferably 0.5% by mass or more, further more preferably 1% by mass or more, even more preferably 5% by mass or more, and further more preferably 10% by mass or more.

In the case of a food product or an oral administration preparation in a liquid form such as a liquid, a syrup, a suspension, an emulsion, an elixir, or a jelly, the total amount of delphinidin-3-rutinoside and cyanidin-3-rutinoside is preferably 0.001% by mass or more, more preferably 0.005% by mass or more, further more preferably 0.01% by mass or more, and even more preferably 0.05% by mass or more.

The dosage amount and dosing regimen of delphinidin-3-rutinoside, cyanidin-3-rutinoside, or the combination thereof can be appropriately determined by those skilled in the art depending on the type, body weight, sex, age, conditions, or other factors of the subject.

The hormone replacement therapy for the symptoms of menopause is a complex mix of risks and benefits, and is suitable for the symptom management in some women; however, it is suggested that it may increase the risk of severe diseases (JAMA, 288(3), 321-333, 2002; JAMA, 310(13), 1353, 2013). Consequently, it is important to improve the indefinite complaint of menopause within the range where a hormone-like action is not expressed. Thus, the dosage amount of delphinidin-3-rutinoside, cyanidin-3-rutinoside, or a combination thereof is such that the total amount of delphinidin-3-rutinoside and cyanidin-3-rutinoside is, for example, preferably 1 mg or more/day, more preferably 10 mg or more/day, further more preferably 25 mg or more/day, and preferably 150 mg or less/day, more preferably 100 mg or less/day, further more preferably 50 mg or less/day per adult (60 kg) from the viewpoint of the ingestion concentration at which the hormone action is not expressed.

The dosage amount of delphinidin-3-rutinoside, cyanidin-3-rutinoside, or the combination thereof is such that the total amount of delphinidin-3-rutinoside and cyanidin-3-rutinoside is from 1 to 150 mg/day, more preferably from 10 to 100 mg/day, and further more preferably from 25 to 50 mg/day per adult (60 kg).

In the present invention, the above dosage is preferably administered or ingested by dividing into, for example, once, twice, three times or more a day. The duration of administration or ingestion is not particularly limited, but is preferably continuous, more preferably 1 week or more, and further more preferably 2 weeks or more.

The timing of administration or ingestion is not particularly limited, but is preferably after breakfast and/or after dinner and before bedtime, and in the case of administration or ingestion after dinner and before bedtime, administration or ingestion is more preferably from 30 minutes to 3 hours before, and further more preferably from 1 hour to 2 hours before the desired bedtime.

The subject to or by which the agent for improving symptoms of menopause of the present invention is administered or ingested is preferably a human or a non-human animal who needs or desires improvement of the symptoms of menopause. More preferably, ingestion or administration by or to a human or a non-human animal in which hot flashes or perspiration is observed as the symptoms of menopause is effective. The human is preferably a human in menopause, and more preferably a woman in menopause.

With respect to the embodiments described above, the present invention further discloses the following aspects.

<1> An agent for improving symptoms of menopause comprising delphinidin-3-rutinoside, cyanidin-3-rutinoside, or a combination thereof as an active ingredient.

<2> A food product for improving symptoms of menopause comprising delphinidin-3-rutinoside, cyanidin-3-rutinoside, or a combination thereof as an active ingredient.

<3> Use of delphinidin-3-rutinoside, cyanidin-3-rutinoside, or a combination thereof for producing an agent for improving symptoms of menopause.

<4> Use of delphinidin-3-rutinoside, cyanidin-3-rutinoside, or a combination thereof for producing a food product for improving symptoms of menopause.

<5> Delphinidin-3-rutinoside, cyanidin-3-rutinoside, or a combination thereof for use in improving symptoms of menopause.

<6> A non-therapeutic use of delphinidin-3-rutinoside, cyanidin-3-rutinoside, or a combination thereof for improving symptoms of menopause.

<7> A method for improving symptoms of menopause, the method comprising administration or ingestion of an effective amount of delphinidin-3-rutinoside, cyanidin-3-rutinoside, or a combination thereof to or by a subject in need thereof.

<8> In <1> to <7>, the symptoms of menopause are preferably dizziness, headaches, sleeplessness, a rush of blood to head, burning sensation, perspiration, palpitation, nausea, constipation, diarrhea, shoulder stiffness, lower back pain, joint pain, frequent urination, drying of the skin and mucous membranes, or eczema, and more preferably hot flashes or perspiration.

<9> In <1> to <8>, delphinidin-3-rutinoside and cyanidin-3-rutinoside are preferably derived from a cassis extract.

<10> In <1> to <9>, a dosage amount of the delphinidin-3-rutinoside, the cyanidin-3-rutinoside, or the combination thereof is such that a total amount of the delphinidin-3-rutinoside and the cyanidin-3-rutinoside is preferably 1 mg or more/day, more preferably 10 mg or more/day, further more preferably 25 mg or more/day, and preferably 150 mg or less/day, more preferably 100 mg or less/day, further more preferably 50 mg or less/day, and the total amount is preferably from 1 to 150 mg/day, more preferably from 10 to 100 mg/day, further more preferably from 25 to 50 mg/day per adult (60 kg).

## Examples

### <Example 1: Purification of cassis anthocyanin>

(1) Crude fraction of cassis extract

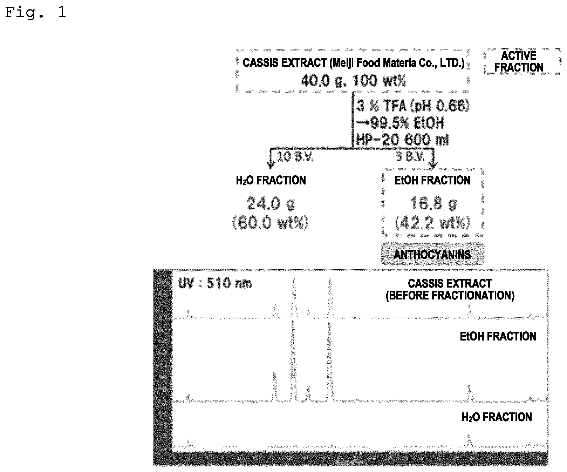

A commercially available cassis extract (cassis polyphenol AC10, the total amount of cassis anthocyanin: 11.8 wt%, manufactured by Meiji Food Materia Co., LTD.) was used and fractionated into anthocyanins and other components. Fractionation by synthetic adsorption resin was carried out with reference to the literature (Polyphenols: Functional Constituents of Medicinal Plants and Foods, CMC Publishing Co., Ltd., 250-251, 2012). As a result, the cassis extract was fractionated into an anthocyanins-containing fraction (EtOH-eluted fraction, four peaks in the HPLC analysis chromatogram were determined to correspond to anthocyanins, 42.2 wt%) and a fraction having higher polarity (HsO-eluted fraction, 60.0 wt%) (see Figure 1).

(2) Anthocyanin purification from EtOH fraction

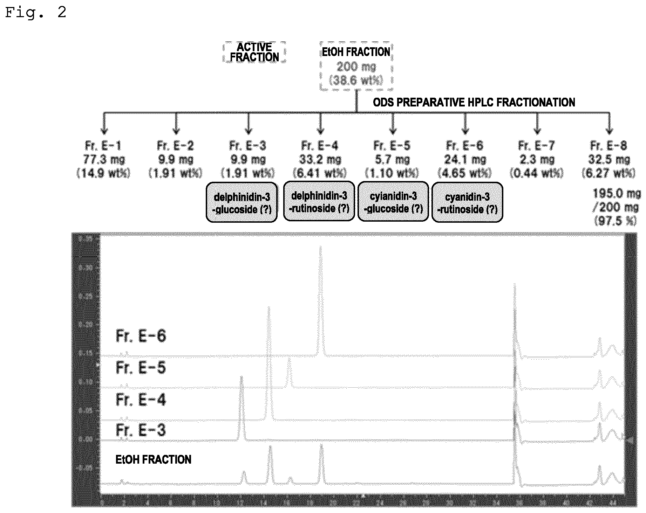

Purification of anthocyanins was carried out from the EtOH fraction (anthocyanins-containing fraction). Fractionation by ODS preparative HPLC was carried out with reference to the conditions of the analytical HPLC. As a result, the EtOH fraction was fractionated into eight fractions in total, that is, 4 anthocyanins-purified fractions and other fractions (Fr. E-1: 14.9 wt%, Fr. E-2: 1.91 wt%, Fr. E-3: 1.91 wt%, Fr. E-4: 6.41 wt%, Fr. E-5: 1.10 wt%, Fr. E-6: 4.65 wt%, Fr. E-7: 0.44 wt%, and Fr. E-8: 6.27 wt%) (see Figure 2).

(3) Identification of purified anthocyanins

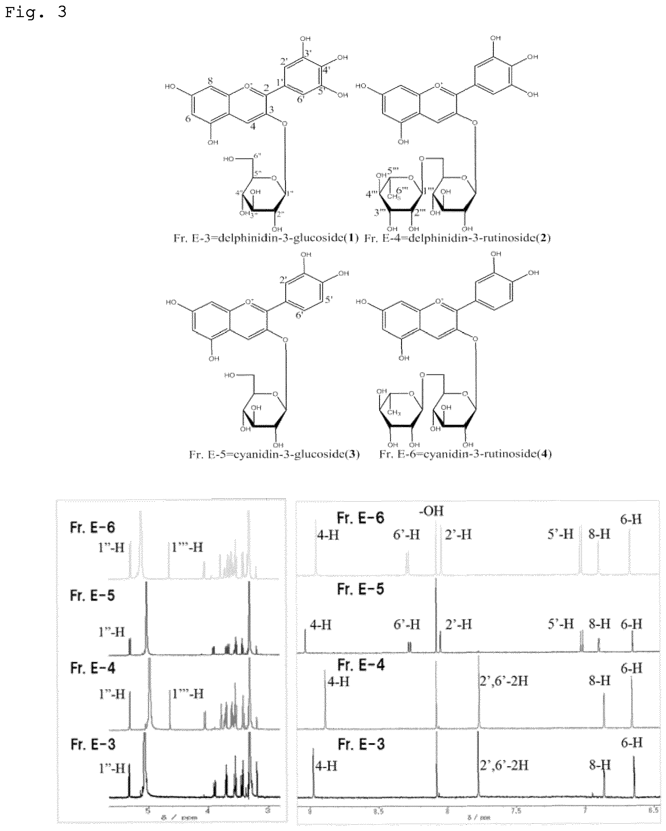

Since four main peaks were separated and purified by preparative HPLC, identification thereof was then carried out. Comparing four peaks with the HPLC analysis chromatogram of the literature (J Agric Food Chem, 50(11), 3228-3231, 2002), it was considered to be highly possible that Fr. E-3 was delphinidin-3-glucoside (1, D3G), Fr. E-4 was delphinidin-3-rutinoside (2, D3R), Fr. E-5 was cyanidin-3-glucoside (3, C3G), and Fr. E-6 was cyanidin-3-rutinoside (4, C3R). Then, $^{1}H$ and $^{13}C$-NMR were measured for each of them, and the attribution of each signal and comparison with those in the literature (J Sep Sci, 40(17), 3506-3512, 2017) confirmed that the peaks were as estimated (see Figure 3).

### <Example 2: Human efficacy test of cassis extract against symptoms of menopause>

(1) Subject

The subjects were 17 healthy women in their 40s to 50s, and the average age of the subjects was 51.5 $\pm$ 1.4 years at the start of the test.

(2) Production of test sample

A commercially available cassis extract containing D3R and C3R (cassis polyphenol AC10, the total amount of cassis anthocyanin: 11.8 wt%, D3R: 5.4 wt%, and C3R: 3.8 wt%, manufactured by Meiji Food Materia Co., LTD.) was formulated into sugarcoated tablets so that the amount of cassis extract per sugarcoated tablet is 100 mg.

(3) Method of ingesting sugarcoated tablets

Sugarcoated tablets (2 tablets/time) were ingested with 150 mL of mineral water twice a day (within 30 minutes after breakfast and from 60 to 30 minutes before bedtime) for 28 days.

(4) Method of evaluating symptoms of menopause

The degree of the symptoms of menopause were evaluated using the Kupperman menopausal index before and after ingestion of the cassis extract-containing sugarcoated tablets. The number of occurrence of hot flashes and perspiration was evaluated using a life diary. The test was carried out by paying attention to the following points during test period.

(Notes)

(i) Try to maintain the normal lifestyle.
(ii) Try to eat as usual.
(iii) Do not change your exercise habit.
(iv) Do not eat and drink too much and do not largely reduce meals (diet).
(v) Do not start taking new supplements.

(5) Statistical test

The obtained results were represented by mean value ± standard error, and the Wilcoxon signed rank test was used for the comparison before and after the ingestion of the cassis extract-containing sugarcoated tablets.

The total score of the Kupperman menopausal index was significantly reduced from 23.7 ± 1.9 to 17.6 ± 2.0, and the severity evaluation scale of the Kupperman menopausal index was also significantly reduced from 2.8 ± 0.2 to 1.9 ± 0.2 ($P < 0.05$) before and after the ingestion of the cassis extract-containing sugarcoated tablets. In addition, the scores of the questions of "hot face (burning sensation)" and "easy to sweat", which are the vasomotor symptoms of the Kupperman menopausal index, were significantly reduced from 6.8 ± 0.8 to 4.5 ± 0.7, and from 6.8 ± 0.8 to 4.0 ± 0.9 ($P < 0.05$), respectively.

The number of occurrences of hot flashes per day was significantly reduced from 1.2 ± 0.3 (times) to 0.5 ± 0.3 (times), and the number of perspirations per day was significantly reduced from 1.2 ± 0.4 (times) to 0.6 ± 0.3 (times) ($P < 0.05$) before and after the ingestion of the cassis extract-containing sugarcoated tablets.

From these results, it was found that the cassis extract had an effect of alleviating the symptoms of menopause, in particular, hot flashes and perspiration, and the ingestion thereof improved the symptoms of menopause, in particular, hot flashes and perspiration.

<Reference Example 1: Evaluation of effect of suppressing TRPV4 activity>

(1) Production of human TRPV4 gene expression vector

Using a primer set consisting of oligonucleotides represented by the base sequences described below which was synthesized with reference to the published human TRPV4 gene sequence using cDNA obtained by reverse transcribing the total RNA extracted from human duodenum-derived cells (Hutu-80 cells, purchased from American Type Culture Collection) as a template, the polymerase chain reaction (PCR) was carried out under the following conditions.

<Primer set>

Forward primer; 5'-CACCATGGCGGATTCCAGCGAAGGCCC-3' (SEQ ID No: 1)
Reverse primer; 5'-CTAGAGCGGGGCGTCATCAGTCC-3' (SEQ ID No: 2)

<PCR conditions>

a) PCR solution composition

| | |
|---|---|
| cDNA (Template) | 15 μL |
| 5x PrimeStar GXL Buffer | 10 μL |

(continued)

| | |
|---|---|
| dNTPs mixture (2.5 mM) | 4 μL |
| PrimeStar GXL DNA Polymerase (Takara Bio Inc.) | 1 μL |
| Forward Primer (10 μM) | 1 μL |
| Reverse Primer (10 μM) | 1 μL |
| Water | 18 μL |

b) Temperature and cycle conditions

95°C 2 min ↓
98°C 10 sec 33 cycles
70°C 2 min

The obtained PCR product was purified using a High Pure PCR Product Purification Kit (manufactured by Roche). Using the purified PCR product and pcDNA3.1 Directional TOPO Expression Kit (manufactured by Invitrogen), a human TRPV4 gene expression vector was produced.

(2) Production of human TRPV4 expression cells

A human cervical cancer-derived cell line (HeLa cells, purchased from American Type Culture Collection) was cultured using a DMEM/F12 medium containing 10% fetal bovine serum (manufactured by Invitrogen). HeLa cells were seeded in a 10 cm cell culture dish at $2 \times 10^5$ cells/dish. After culturing for 2 days, the cells were transfected with the human TRPV4 gene expression vector (7.7 μg) produced in the above (1) using TransIT-HeLaMONSTER Transfection Kit (manufactured by Mirus) and cultured for 1 day.

The cells were detached using Detachin (manufactured by Genlantis Inc.), seeded in a DMEM/F12 medium containing 10% fetal bovine serum on a 96 well Optical bottom plate (manufactured by Nunc A/S) at a cell density of $2.0 \times 10^4$ cells/90 μL/well, and further cultured for 1 day.

(3) Measurement of intracellular calcium ion influx activity

Measurement of the intracellular calcium ion influx activity was carried out using Calcium Kit II-fluo 4 (trade name, DOJINDO). 90 μL/well of a loading buffer containing Fluo4-AM was added to the human TRPV4 expression cells produced in the above (2), and incubated at 37°C for 1 hour. Thereafter, the fluorescence intensity (excitation wavelength: 488 nm, fluorescence wavelength: 524 nm) was measured at 37°C every two seconds using a fluorescence plate reader (FDSS/μCELL Functional Drug Screening System C13299, manufactured by Hamamatsu Photonics K.K.). After 30 seconds from the start of the measurement, each of GSK1016790a (manufactured by Sigma) which is the TRPV4 agonist, and the cassis extract, or D3R, C3R, D3G, or C3G prepared in the above Production Example as the sample was diluted with a dilution buffer of the measurement kit, 20 μL/well of a mixed solution thereof (mixed immediately before addition) was added, and the change of fluorescence intensity was measured every two seconds until 300 seconds. GSK1016790a was added so that the final concentration was 10 nM, and the evaluation concentration of each anthocyanin was set based on the content in the cassis extract.

The TRPV4 activity of the sample was calculated by the following equation assuming that the calcium ion influx rate by the treatment of GSK1016790a which is the TRPV4 agonist was 100%.

(Equation)

$$\text{Calcium ion influx rate (\%)} = [(F300/F0) - (F300C2/F0C2)]/[(F300C1/F0C1) - (F300C2/F0C2)] \times 100$$

F300: The fluorescence intensity of the well into which GSK1016790a and the sample were added after 300 seconds from the start of the measurement

F300C1: The fluorescence intensity of the well into which GSK1016790a and a solvent were added after 300 seconds from the start of the measurement

F300C2: The fluorescence intensity t of the well into which only a solvent was added after 300 seconds from the start of the measurement
F0: The fluorescence intensity of the same well as F300 immediately after the start of the measurement
FOC1: The fluorescence intensity of the same well as F300C1 immediately after the start of the measurement
F0C2: The fluorescence intensity of the well into which only a solvent was added immediately after the start of the measurement

The calcium ion influx rate in the case of adding the sample was tested using the Dunnett's test by comparing with that in the case of adding GSK1016790a + a solvent. The results in the case of adding the cassis extract, or D3R, C3R, D3G, or C3G are shown in the table as mean value ± standard error (in the table, GSK1016790a, the cassis extract, the ethanol fraction of the cassis extract, the water fraction of the cassis extract, and the mixture of four components, D3R, C3R, D3G, and C3G (D3R: 45.6 wt%, C3R: 33.0 wt%, D3G: 13.6 wt%, and C3G: 7.8 wt%) were respectively abbreviated as GSK, CaE, EtOH, $H_2O$, and MIX).

[Table 1]

| | Intracellular $Ca^{2+}$ increase rate (%) |
|---|---|
| GSK 10 nM + Solvent | 100.0 ± 1.6 |
| GSK 10 nM + CaE 10 μg/mL | 75.9 ± 1.0 (P<0.01) |
| GSK 10 nM + CaE 100 μg/mL | 72.7 ± 2.8 (P<0.001) |
| GSK 10 nM + EtOH 4.2 μg/mL | 74.4 ± 1.9 (P<0.001) |
| GSK 10 nM + EtOH 42 μg/mL | 73.0 ± 2.1 (P<0.001) |
| GSK 10 nM + $H_2O$ 6 μg/mL | 97.8 ± 3.7 |
| GSK 10 nM + $H_2O$ 60 μg/mL | 99.4 ± 7.2 |
| | n=3, Mean ± SE |
| | Intracellular $Ca^{2+}$ increase rate (%) |
| GSK 10 nM + Solvent | 100.0 ± 1.6 |
| GSK 10 nM + MIX 1 μg/mL | 70.9 ± 4.1 (P<0.001) |
| GSK 10 nM + MIX 10 μg/mL | 69.8 ± 3.0 (P<0.001) |
| GSK 10 nM + D3R 0.46 μg/mL | 89.5 ± 2.0 |
| GSK 10 nM + D3R 4.6 μg/mL | 78.8 ± 1.2 (P<0.01) |
| GSK 10 nM + C3R 0.35 μg/mL | 95.3 ± 4.2 |
| GSK 10 nM + C3R 3.5 μg/mL | 86.6 ± 5.1 (P=0.07) |
| GSK 10 nM + D3G 0.14 μglmL | 101.4 ± 2.7 |
| GSK 10 nM + D3G 1.4 μg/mL | 100.4 ± 3.2 |
| GSK 10 nM + C3G 0.05 μg/mL | 94.4 ± 3.1 |
| GSK 10 nM + C3G 0.5 μg/mL | 101.5 ± 4.1 |
| n=3, Mean ± SE | |

As seen from Table 1, the cassis extract and the ethanol fraction of the cassis extract significantly reduced the calcium ion influx. In addition, the mixture of four main components of the ethanol fraction, D3R, C3R, D3G, and C3G (MIX) significantly reduced the calcium ion influx. In particular, D3R significantly reduced the calcium ion influx and C3R tended to reduce the calcium ion influx.

From these results, the TRPV4 activity is suppressed by applying the cassis extract, D3R, or C3R. This demonstrates that the cassis extract, D3R, or C3R is effective for the suppression of the TRPV4 activity.

Further, this demonstrates that the cassis extract, D3R, or C3R having the effect of suppressing the TRPV4 activity is effective for the improvement of the symptoms of menopause.

## Claims

1. An agent for improving symptoms of menopause comprising delphinidin-3-rutinoside, cyanidin-3-rutinoside, or a combination thereof as an active ingredient.

2. The agent for improving symptoms of menopause according to claim 1, wherein the symptoms of menopause are hot flashes and perspiration.

3. The agent for improving symptoms of menopause according to claim 1 or 2, wherein the delphinidin-3-rutinoside and the cyanidin-3-rutinoside are derived from a cassis extract.

4. The agent for improving symptoms of menopause according to any one of claims 1 to 3, wherein the delphinidin-3-rutinoside, the cyanidin-3-rutinoside, or the combination thereof is administered or ingested in an amount such that a total amount of the delphinidin-3-rutinoside and the cyanidin-3-rutinoside is from 1 to 150 mg/day per adult (60 kg).

5. A food product for improving symptoms of menopause comprising delphinidin-3-rutinoside, cyanidin-3-rutinoside, or a combination thereof as an active ingredient.

6. The food product for improving symptoms of menopause according to claim 4, wherein the symptoms of menopause are hot flashes and perspiration.

7. The food product for improving symptoms of menopause according to claim 4 or 5, wherein the delphinidin-3-rutinoside and the cyanidin-3-rutinoside are derived from a cassis extract.

8. The food product for improving symptoms of menopause according to any one of claims 5 to 7, wherein the delphinidin-3-rutinoside, the cyanidin-3-rutinoside, or the combination thereof is administered or ingested in an amount such that a total amount of the delphinidin-3-rutinoside and the cyanidin-3-rutinoside is from 1 to 150 mg/day per adult (60 kg).

9. Use of delphinidin-3-rutinoside, cyanidin-3-rutinoside, or a combination thereof for producing an agent for improving symptoms of menopause.

10. Use of delphinidin-3-rutinoside, cyanidin-3-rutinoside, or a combination thereof for producing a food product for improving symptoms of menopause.

11. The use according to claim 9 or 10, wherein the symptoms of menopause are hot flashes and perspiration.

12. The use according to any one of claims 9 to 11, wherein the delphinidin-3-rutinoside and the cyanidin-3-rutinoside are derived from a cassis extract.

13. The use according to any one of claims 9 to 12, wherein the delphinidin-3-rutinoside, the cyanidin-3-rutinoside, or the combination thereof is administered or ingested in an amount such that a total amount of the delphinidin-3-rutinoside and the cyanidin-3-rutinoside is from 1 to 150 mg/day per adult (60 kg).

14. Delphinidin-3-rutinoside, cyanidin-3-rutinoside, or a combination thereof for use in improving symptoms of menopause.

15. The delphinidin-3-rutinoside, the cyanidin-3-rutinoside, or the combination thereof according to claim 14, wherein the symptoms of menopause are hot flashes and perspiration.

16. The delphinidin-3-rutinoside, the cyanidin-3-rutinoside, or the combination thereof according to claim 14 or 15, wherein the delphinidin-3-rutinoside and the cyanidin-3-rutinoside are derived from a cassis extract.

17. The delphinidin-3-rutinoside, the cyanidin-3-rutinoside, or the combination thereof according to any one of claims 14 to 16, wherein the delphinidin-3-rutinoside, the cyanidin-3-rutinoside, or the combination thereof is administered or ingested in an amount such that a total amount of the delphinidin-3-rutinoside and the cyanidin-3-rutinoside is from 1 to 150 mg/day per adult (60 kg).

**18.** A non-therapeutic use of delphinidin-3-rutinoside, cyanidin-3-rutinoside, or a combination thereof for improving symptoms of menopause.

**19.** The non-therapeutic use according to claim 18, wherein the symptoms of menopause are hot flashes and perspiration.

**20.** The non-therapeutic use according to claim 18 or 19, wherein the delphinidin-3-rutinoside and the cyanidin-3-rutinoside are derived from a cassis extract.

**21.** The non-therapeutic use according to any one of claims 18 to 20, wherein the delphinidin-3-rutinoside, the cyanidin-3-rutinoside, or the combination thereof is administered or ingested in an amount such that a total amount of the delphinidin-3-rutinoside and the cyanidin-3-rutinoside is from 1 to 150 mg/day per adult (60 kg).

**22.** A method for improving symptoms of menopause, the method comprising administration or ingestion of an effective amount of delphinidin-3-rutinoside, cyanidin-3-rutinoside, or a combination thereof to or by a subject in need thereof.

**23.** The method according to claim 22, wherein the symptoms of menopause are hot flashes and perspiration.

**24.** The method according to claim 22 or 23, wherein the delphinidin-3-rutinoside and the cyanidin-3-rutinoside are derived from a cassis extract.

**25.** The method according to any one of claims 22 to 24, wherein the delphinidin-3-rutinoside, the cyanidin-3-rutinoside, or the combination thereof is administered or ingested in an amount such that a total amount of the delphinidin-3-rutinoside and the cyanidin-3-rutinoside is from 1 to 150 mg/day per adult (60 kg).

Fig. 1

CASSIS EXTRACT (Meiji Food Materia Co., LTD.)
40.0 g, 100 wt%
ACTIVE FRACTION
3 % TFA (pH 0.66)
→99.5% EtOH
HP-20 600 ml
10 B.V.
3 B.V.
H2O FRACTION
24.0 g
(60.0 wt%)
EtOH FRACTION
16.8 g
(42.2 wt%)
ANTHOCYANINS
UV : 510 nm
CASSIS EXTRACT
(BEFORE FRACTIONATION)
EtOH FRACTION
H2O FRACTION

Fig. 2

ACTIVE FRACTION
EtOH FRACTION
200 mg
(38.6 wt%)
ODS PREPARATIVE HPLC FRACTIONATION
Fr. E-1
77.3 mg
(14.9 wt%)
Fr. E-2
9.9 mg
(1.91 wt%)
Fr. E-3
9.9 mg
(1.91 wt%)
Fr. E-4
33.2 mg
(6.41 wt%)
Fr. E-5
5.7 mg
(1.10 wt%)
Fr. E-6
24.1 mg
(4.65 wt%)
Fr. E-7
2.3 mg
(0.44 wt%)
Fr. E-8
32.5 mg
(6.27 wt%)
delphinidin-3 -glucoside (?)
delphinidin-3 -rutinoside (?)
cyianidin-3 -glucoside (?)
cyanidin-3 -rutinoside (?)
195.0 mg
/200 mg
(97.5 %)
Fr. E-6
Fr. E-5
Fr. E-4
Fr. E-3
EtOH FRACTION

Fig. 3

Fr. E-3=delphinidin-3-glucoside(**1**) Fr. E-4=delphinidin-3-rutinoside(**2**)

Fr. E-5=cyanidin-3-glucoside(**3**) Fr. E-6=cyanidin-3-rutinoside(**4**)

Fr. E-6
1"-H
1'''-H
Fr. E-5
1"-H
Fr. E-4
1"-H
1'''-H
Fr. E-3
1"-H
5
4
3
δ / ppm

Fr. E-6
-OH
4-H
6'-H
2'-H
5'-H
8-H
6-H
Fr. E-5
4-H
6'-H
2'-H
5'-H
8-H
6-H
Fr. E-4
4-H
2',6'-2H
8-H
6-H
Fr. E-3
4-H
2',6'-2H
8-H
6-H
9
8.5
8
7.5
7
6.5

## INTERNATIONAL SEARCH REPORT

International application No.
PCT/JP2021/015706

A. CLASSIFICATION OF SUBJECT MATTER
Int. Cl. A61K31/7048(2006.01)i, A61K36/185(2006.01)i, A61P9/00(2006.01)i, A61P15/12(2006.01)i
FI: A61K31/7048 ZNA, A61P9/00, A61P15/12, A61K36/185
According to International Patent Classification (IPC) or to both national classification and IPC

B. FIELDS SEARCHED
Minimum documentation searched (classification system followed by classification symbols)
Int. Cl. A61K31/7048, A61K36/185, A61P9/00, A61P15/12

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | CN 109090414 A (TIANJIN UNIVERSITY OF SCIENcE & TECHNOLOGY) 28 December 2018, claims, paragraphs [0003], [0004], examples 1-4, animal experimentation 1, claims, paragraphs [0003], [0004], examples 1-4, animal experimentation 1 | 1-25<br>2, 6, 11, 15, 19, 23 |
| X<br><br>Y | NANASHIMA, Naoki et al. Blackcurrant extract with phytoestrogen activity alleviates hair loss in ovariectomized rats, Molecules, 2019, vol. 24, no. 7, Article No. 1272, pp. 1-11, in particular, abstract, 1. Introduction, 2. Results and Discussion, 3. Materials and Methods, 4. Conclusions, in particular, abstract, 1. Introduction, 2. Results and Discussion, 3. Materials and Methods, 4. Conclusions | 1, 3-5, 7-10, 12-14, 16-18, 20-22, 24, 25<br>2, 6, 11, 15, 19, 23 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26.05.2021 | 08.06.2021 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No. PCT/JP2021/015706

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y | HORIE, Kayo et al. Phytoestrogenic effects of blackcurrant anthocyanins increased endothelial nitric oxide synthase (eNOS) expression in human endothelial cells and ovariectomized rats, Molecules, 2019, vol. 24, no. 7, article No. 1259, pp. 1-12, in particular, abstract, 1. Introduction, 2. Results and Discussion, 3. Materials and Methods, 4. Conclusions, in particular, abstract, 1. Introduction, 2. Results and Discussion, 3. Materials and Methods, 4. Conclusions | 1, 3-5, 7-10, 12-14, 16-18, 20-22, 24, 25<br>2, 6, 11, 15, 19, 23 |
| X<br><br>Y | NANASHIMA, Naoki et al. Blackcurrant anthocyanins increase the levels of collagen, elastin. and hyaluronic acid in human skin fibroblasts and ovariectomized rats, Nutrients, 2018, vol. 10, no. 4, article No. 495, pp. 1-15, in particular, abstract, 1. Introduction, 2. Materials and Methods, 3. Results, 4. Discussion, 5. Conclusions, in particular, abstract, 1. Introduction, 2. Materials and Methods, 3. Results, 4. Discussion, 5. Conclusions | 1, 3-5, 7-10, 12-14, 16-18, 20-22, 24, 25<br>2, 6, 11, 15, 19, 23 |
| X | ALINEZHAD, G. et al. Evaluation effect of blackcurrant on testis tissue in Wistar rat. International Journal of Fertility and Sterility, 2016, vol. 10, Supp. 1, p. 34, abstract no. P-2, in particular, Background, Materials and Methods, Results, Conelusions | 1, 3-5, 7-10, 12-14, 16-18, 20-22, 24, 25 |
| X | JP 2008-110933 A (FOUR LEAF JAPAN KK) 15 May 2008, claims, paragraphs [0010], [0014], experimental examples | 1-25 |
| P, X | JP 2020-186215 A (HIROSAKI UNIVERSITY) 19 November 2020, claims, examples | 1-25 |
| P, A | US 2020/0276255 A1 (NINE B CO., LTD.) 03 September 2020, claims, examples | 1-25 |
| A | WO 01/01798 A1 (MEIJI SEIKA KAISHA, LTD.) 11 January 2001, claims, examples | 1-25 |

Form PCT/ISA/210 (continuation of second sheet) (January 2015)

**INTERNATIONAL SEARCH REPORT**
Information on patent family members

International application No.
PCT/JP2021/015706

| Patent Documents referred to in the Report | Publication Date | Patent Family | Publication Date |
|---|---|---|---|
| CN 109090414 A | 28.12.2018 | (Family: none) | |
| JP 2008-110933 A | 15.05.2008 | (Family: none) | |
| JP 2020-186215 A | 19.11.2020 | (Family: none) | |
| US 2020/0276255 A1 | 03.09.2020 | (Family: none) | |
| WO 01/01798 A1 | 11.01.2001 | JP 3455528 B2<br>US 2010/0015065 A1<br>claims, examples<br>EP 1208755 A1<br>CA 2373825 A1<br>KR 10-0547497 B1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6075830 B
- JP 5931633 B
- JP 4077149 B


### Non-patent literature cited in the description


- *Molecules,* 2018, vol. 23 (1), 74
- menopausal medicine. The guidebook on Women's health care. 2014
- *JAMA,* 2002, vol. 288 (3), 321-333
- *JAMA,* 2013, vol. 310 (13), 1353
- Polyphenols: Functional Constituents of Medicinal Plants and Foods. CMC Publishing Co., Ltd, 2012, 250-251
- *J Agric Food Chem,* 2002, vol. 50 (11), 3228-3231
- *J Sep Sci,* 2017, vol. 40 (17), 3506-3512